(19) Europäisches Patentamt
European Patent Office
Office européen des brevets

(11) EP 1 853 633 B1

(12) **EUROPEAN PATENT SPECIFICATION**

(45) Date of publication and mention of the grant of the patent:
**14.09.2016 Bulletin 2016/37**

(21) Application number: **06736908.2**

(22) Date of filing: **27.02.2006**

(51) Int Cl.:
***C08B 11/08*** *(2006.01)*

(86) International application number:
**PCT/US2006/007663**

(87) International publication number:
**WO 2006/094211 (08.09.2006 Gazette 2006/36)**

(54) **WATER-SOLUBLE, LOW SUBSTITUTION HYDROXYETHYLCELLULOSE, DERIVATIVES THEREOF, PROCESS OF MAKING, AND USES THEREOF**

WASSERLÖSLICHE HYDROXYETHYLCELLULOSE MIT NIEDRIGER SUBSTITUTION, DERIVATE DAVON, HERSTELLUNGSVERFAHREN UND VERWENDUNG

HYDROXYÉTHYLCELLULOSE HYDROSOLUBLE À FAIBLE DEGRÉ DE SUBSTITUTION, DÉRIVÉS, PROCÉDÉ DE FABRICATION ET UTILISATIONS CORRESPONDANTS

(84) Designated Contracting States:
**AT BE BG CH CY CZ DE DK EE ES FI FR GB GR HU IE IS IT LI LT LU LV MC NL PL PT RO SE SI SK TR**

(30) Priority: **02.03.2005 US 657963 P**

(43) Date of publication of application:
**14.11.2007 Bulletin 2007/46**

(73) Proprietor: **Hercules Incorporated**
**Wilmington, DE 19808 (US)**

(72) Inventors:
- **ARISZ, Petrus, Wilhelmus, Franciscus**
**1095 TH Amsterdam (NL)**
- **LUSVARDI, Kate, M.**
**Chadds Ford, Pennsylvania 19317 (US)**

(74) Representative: **Hoffmann Eitle**
**Patent- und Rechtsanwälte PartmbB**
**Arabellastraße 30**
**81925 München (DE)**

(56) References cited:
**US-A- 3 709 876**
**US-A- 4 024 335**
**US-B1- 6 482 940**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 853 633 B1

Printed by Jouve, 75001 PARIS (FR)

## Description

### FIELD OF INVENTION

The present invention relates to a process for making cellulose ether compositions. More specifically, this invention relates to a process for making water-soluble hydroxyethylcelluloses (HECs) having a hydroxyethyl molar substitution (HE-MS) from 0.7 to 1.3.

### BACKGROUND OF THE INVENTION

Hydroxyethylcellulose (HEC) is cellulose ether usually made by reacting alkali cellulose with ethylene oxide (EO). In general the molar ratio of EO to the anhydroglucose units of cellulose is higher than 1.5 to provide adequate water-solubility to the cellulose backbone. HEC is a water-soluble/water-swellable polymer that generally is used to viscosity aqueous media of functional systems such as personal care and household products, paints, construction material products, paper coatings, oilfield media, emulsions, latex components, etc. Furthermore, high molecular weight HEC is used in the pharmaceutical industry as an excipient to provide a swellable diffusion barrier in controlled release applications.

In commercial HECs that are made by a single-stage ethoxylation of cellulose, the hydroxyethylene substituents are nearly randomly distributed among the anhydroglucose segments of the polymer. Examples of prior art that disclose the preparation of HEC are US Patents 2,572,039, 2,682,535, 2,744,894, and 3,131,177. Another commercial HEC product is a more highly substituted HEC in which the ethylene oxide is reacted in two-steps thereby reducing the amount of unsubstituted anhydroglucose units. This results in the formation of a cellulose derivative that is less susceptible to enzymatic degradation, i.e. enhanced resistance to biodegradation. Examples of prior art that disclose the preparation of this type of HEC are US Patent 3,131,176, Canadian Patent 1014289, and US Patent Application US 2005/0139130 A1. The solution viscosities of HECs with these types of EO substitution patterns usually depend on the molecular weight of the cellulose backbone.

U.S. Patent Application Serial No. 11/353,621, entitled "Water-Soluble, Low Substitution Hydroxyethylcellulose, Derivatives Thereof, Process Of Making, And Uses Thereof," filed February 14, 2006 discloses non-uniformly substituted ("blocky") hydroxyethylcelluloses (HECs) and derivatives thereof that show associative thickening properties that are unknown in commercial HEC products. These blocky HEC products are characterized by a novel parameter called the unsubstituted trimer ratio (U3R) which is defined as the ratio of the molar fraction of unsubstituted trimers to the molar fraction of the most abundant class of (hydroxyethyl-substituted) trimers. The U3R of blocky products is greater than 0.21 for the HE-MS range of 1.3 and 5.0.

Furthermore, HECs can be modified with additional substituents to improve functionality. For example, US Patent No. 4,228,277 discloses the use of long chain alkyl modifiers having 10 to 24 carbon atoms. Another example of a modified HEC is disclosed in US Patent No. 4,826,970 that describes a carboxymethyl hydrophobically modified hydroxyethyl cellulose ether derivative (CMHMHEC) that is used as thickeners and protective colloids in water based protective coating compositions. US Patent No 4,904,772 discloses a water-soluble HEC derivative that has a mixed hydrophobe having two or more hydrophobic radicals having 6 to 20 carbons whereby one of the hydrophobic radicals has a carbon chain length that is at least two carbon atoms longer than that of the other hydrophobic radical. US Patent 4,663,159 discloses a water-soluble, cationic hydroxyethyl cellulose.

Commercial HEC products are the thickeners of choice in many industries because they provide the desired rheology and thickening efficiency. Notwithstanding, a need always exists for an HEC-based rheology modifier that would be more efficient in thickening aqueous systems and interact more strongly with components in the system and/or with itself so that additional desired rheological properties can be achieved.

### SUMMARY OF THE INVENTION

The present invention is directed to a process for making a new class of lowly substituted HECs in which the EO is distributed extremely uniformly along the backbone of the cellulose in order to render it water-solubfe. This unique class of HEC is water-soluble unlike other classes of HEC in the prior art with similar hydroxyethyl molar substitution (HE-MS) arid cellulose molecular weight. These lowly substituted HECs can be further modified with hydrophobic, cationic, or anionic reagents.

An advantage of this product is that it provides a much higher solution viscosity than regular commercial HEC at similar concentrations and molecular weight. Consequently, a lesser amount of the HEC obtained in the present invention can produce comparable or better viscosity relative to analogous commercial HECs of similar molecular weight. Furthermore, it has been surprisingly found that HECs and derivatives thereof obtained by the present invention are efficient thickeners and suspending agents in low-water activity solutions such as those that contain high concentrations

of salt or those that contain a fraction of a miscible organic solvent. There is a need for universal thickeners that function in the broad spectrum of aqueous systems as cellulosics, natural gums, and synthetic carbomers often do not dissolve or function in solutions that have only a low level of free water available.

The present invention is directed to a process for making water-soluble HECs that have hydroxyethyl groups that are uniformly distributed on the cellulose backbone, wherein the ratio of unsubstituted anhydroglucose trimers to the most frequently occurring substituted anhydroglucose trimers (U3R) is less than 0.21 and the hydroxyethyl molar substitution is in the range of 0.7 to 1.3.

The slurry process of the present invention for making the above mentioned HEC composition comprises

A) mixing and reacting cellulose, water and alkali in an organic solvent for a sufficient time and at a sufficient temperature in order to form an alkali cellulose mixture, wherein the water to anhydroglucose (AGU) molar ratio is in the range of 5 to 35 and the alkali to AGU molar ratio is greater than 1.6,
B) adding a sufficient amount of ethylene oxide to produce the desired hydroxyethyl molar substitution (HE-MS)
C) adding a sufficient amount of acid continuously in order to reduce the alkali to AGU molar ratio to less than 0.4 and greater than 0.04, while reacting the ethylene oxide with the alkali cellulose at a sufficient temperature and for a sufficient time to form a water soluble HEC product with a HE-MS in the range of 0.7 to 1.3 and a U3R of equal to or less than 0.21.

The HEC product prepared by the above mentioned process can optionally be further reacted with at least one other denvatizing reagent to form a modified HEC product.

Likewise, the HEC or modified HEC product, optionally, can further be reacted with a viscosity reducing agent.

The present invention is also related to a functional system composition including the water-soluble, low HE-MS HEC composition or derivatives thereof.

## BRIEF DESCRIPTION OF THE DRAWING

Figure 1 shows a bar graph of the ethylene oxide distribution profile of a HEC polymer.

## DETAILED DESCRIPTION OF THE INVENTION

It has been surprisingly found that low HE-MS, water-soluble HECs can produce unique rheology that has not been noted prior to this invention and can be prepared using a continuous caustic reduction during the hydroxyethylation manufacturing process.

The present invention allows making water-soluble; low HE-MS HECs and modified HECs (nonionic, anionic, and cationic) in which a large fraction of the anhydroglucose units (AGU) in the molecule are substituted with ethylene oxide (EO). The features that differentiate these HECs from prior art are high water solubility at a HE-MS that is in the range of 0.7 to 1.3; and a structural parameter, the unsubstituted trimer ratio (U3R) that is less than 0.21. This unique class of soluble HECs while nearly uniformly hydroxyethylated shows surprising associative behavior through hydrogen bonding due to the very low HE-MS. As a result, these HECs exhibit significantly higher solution viscosities as compared to other classes of HECs with similar HE-MS (hydroxyethyl molar substitution) and cellulose molecular weight. Furthermore, they show excellent viscosifying power in salt-containing solutions and lean solvents, whereas commercial HECs have difficulty hydrating.

In accordance with the present invention, the water-soluble low HE-MS HEC composition can be further modified with one or more nonionic, anionic, and cationic substituents or mixtures thereof. The substituents are attached to the HEC backbone via an ether, ester, or urethane linkage.

When the substituents have nonionic chemical functionality, the substituents have the formula:

-R, or -A-R, wherein A is

$CH_2$-CH(OH),

$CH_2$-CH(OH)-$CH_2$,

($CH_2$-$CH_2$-O)$_n$ where n = 1 - 100,

$CH_2$-CH(OH)-$CH_2$O-($CH_2$-$CH_2$-O)$_n$ where n = 1 - 100,

CH(R)-C(O)-$CH_2$, and

R is selected from one of the following groups:

i) an acyclic or cyclic, saturated or unsaturated, branched or linear hydrocarbon moiety having 1 to 30 carbon atoms,
ii) an acyclic or cyclic, saturated or unsaturated, branched or linear heterohydrocarbon moiety having 1 to 30 carbon atoms and one of more oxygen, nitrogen, or silicone atoms,
iii) an acyclic or acyclic, saturated or unsaturated, branched or linear hydrocarbon moiety having 1 to 30 carbon atoms and one or more aromatic hydrocarbon groups,
iv) an acyclic or cyclic, saturated or unsaturated, branched or linear heterohydrocarbon moiety having 1 to 30 carbon atoms and one or more oxygen, nitrogen, or silicone atoms and one or more aromatic groups, and
v) an acyclic or cyclic, saturated or unsaturated, branched or linear, heterohydrocarbon moiety having 1 to 30 carbon atoms and one or more oxygen, nitrogen, or silicone atoms and one or more heteroaromatic groups containing one or more oxygen, nitrogen, or silicone groups.

Based on the formula R above, the substituents may be selected from alkyl, alkenyl, alkynyl, aryl, alkyl aryl, aryl alkyl, alkenyl aryl, aryl alkenyl, or mixtures thereof, having when possible, from 1 to 30 carbon atoms.

When the substituents have anionic chemical functionality, the anionic chemical functionality can be carboxylate, sulfate, sulfonate, phosphate, phosphonate or mixtures thereof. More specific examples of this functionality are carboxymethyl, sulfoethyl, phosphonomethyl, and mixtures thereof.

When the substituerits have cationic chemical functionality, the substituents have the formula $R^1R^2R^3R^4N^+$ ($A^-$), where $R^1$ is . $-CH_2-CHOH-CH_2-$= or $-CH_2-CH_2-$, and $R^2$, $R^3$, $R^4$ are each independently selected from an alkyl or aryl alkyl group having 1 to 20 carbon atoms, and $A^-$ is a halide, sulfate, phosphate, or tetrafluoroborate ion.

More specifically, the cationic substituents can be selected from 2-hydroxpropyltrimethylammonium chloride, 2-hydroxypropyldodecyldimethylammonium chloride, 2-hydroxypropylcocoalkyldimethylammonium chloride, 2-hydroxypropyloctadecyldimethylammonium chloride and mixtures thereof.

Another important cationic group that can be used in this invention is the group derived from the grafting reaction of diallyldimethylammonium chloride with HEC or its derivatives.

In accordance with the present invention, more specific modified hydroxyethylcellulose are methyl hydroxyethylcellulose, ethyl hydroxyethylcellulose, octyl hydroxyethylcellulose, cetyl hydroxyethylcellullose, cetoxy-2-hydroxypropyl hydroxyethylcellulose, butoxy-2-hydroxypropyl hydroxyethylcellulose, butoxy-2-hydroxypropyl cetyl hydroxyethylcellulose, butoxy-2-hydroxypropyl cetoxy-2-hydroxyethylcellulose, carboxymethyl hydroxyethylcellulose, carboxymethyl ethyl hydroxyethylcellulose, carboxymethyl octyl hydroxyethylcellulose, carboxymethyl cetyl hydroxyethylcellulose, carboxymethyl cetoxy-2-hydroxypropylcellulose, carboxymethyl butoxy-2-hydroxyethylcellulose, sulfoethyl hydroxyethylcellulose, sulfoethyl ethyl hydroxyethylcellulose, sulfoethyl cetyl hydroxyethylcellulose, sulfoethyl cetoxy-2-hydroxypropylcellulose, 2- hydroxypropyltrimethylammonium chloride hydroxyethylcellulose, 2-hydroxypropyltrimethylammonium chloride ethyl hydroxyethylcellulose, 2-hydroxypropyltrimethylammonium chloride butoxy-2-hydroxypropyl hydroxyethylcellulose, 2-hydroxypropyltrimethylammonium chloride octyl hydroxyethylcellulose, 2-hydroxypropyltrimethylammonium chloride cetyl hydroxyethylcellulose, 2-hydroxypropyltrimethylammonium chloride cetoxy-2-hydroxypropyl hydroxyethylcellulose, 2-hydroxypropyllauryldimethylammonium chloride hydroxyethylcellulose, 2- hydroxypropyltrimethylammonium chloride 2-hydroxypropyllauryldimethylammonium chloride hydroxyethylcellulose, diallyldimethylammonium chloride grafted hydroxyethylcellulose, diallyldimethylammonium chloride grafted cetyl hydroxyethylcellulose.

The lowly hydroxyethylated water soluble HECs can be prepared by completely opening up the cellulose fiber with high initial caustic level (AC1) and then "quenching" continuously to a low caustic level (AC2) during the hydroxyethylation reaction. This process drives a more uniform substitution so as to render high water solubility at low HE-MS. In accordance with the present invention, the process requires mixing and reacting cellulose with water and alkali in an organic solvent wherein the molar ratio of alkali to anhydroglucose (AGU) is greater than 1.6 and the molar ratio of water to AGU is 5 - 35. After 1 hour at 20° C, a high base content alkali cellulose is formed. Subsequently, EO is added to the reaction mixture so that upon reacting, the HE-MS of the final HEC product will be 0.7 to 1.3. Next, the high base content alkall cellulose is continuously neutralized with a sufficient amount of an acid to reduce the alkali content to an alkali to AGU molar ratio between 0.4 and 0.04 while simultaneously reacting the ethylene oxide with the alkali cellulose at 60° C to form a water-soluble hydroxyethylcellulose product. The acid can be added over 30 to 90 minutes during the hydroxyethylation. Upon completion.of the hydroxyethylation, the product can be further modified with nonionic, anionic or cationic reagents. In addiction, the product can be viscosity reduced, purified, dried, and ground as known to those skilled in the art.

In the slurry process of the present invention, organic solvent used in this process is selected from ethanol, isopropanol, tert-butanol, acetone, methyl ethyl ketone, dimethoxyethane, or mixtures thereof. This slurry process uses

alkalis that are selected from lithium hydroxide, sodium hydroxide, potassium hydroxide, and mixtures thereof. The raw cellulose starting material used in the process for making the low HE-MS, water soluble HECs can be cotton tinters, wood pulps, or mixtures thereof.

The water-soluble HEC compositions mentioned above can be optionally further reacted with at least another derivatizing reagent to form a modified hydroxyethylcellulose composition. The derivatizing reagent used to make this modified hydroxyethylcellulose composition can be nonionic, cationic, or anionic organic compounds or mixtures thereof. These organic compounds capable of reacting with the hydroxyls groups of the HEC can be halides, epoxides, glycidyl ethers, carboxylic acids, isocyanates, or mixtures thereof.

The HEC or derivatives thereof made by the slurry processes mentioned above can be further reacted with a viscosity reducing agent, such as peroxide, persulfate, peracid, salt of halide oxo acids, oxygen, or ozone. This enables a person using this process to modify the final product to the desired viscosity or other properties for the desired end use.

The process and process conditions determine how the EO is distributed along the cellulose backbone. Products obtained by the invention are characterized and can be differentiated from HECs made by prior art by reducing the low HE-MS polymer down to monomers and oligomers and measuring the degree of unsubstituted oligomers, more specifically unsubstituted trimers. A parameter called the unsubstituted trimer ratio (U3R) can be defined as the ratio of the molar fraction of unsubstituted trimers to the molar fraction of the most abundant class of (hydroxyethyl-substituted) trimers, with $0 \leq U3R \leq 1.0$. U3R is measured by a mass specfirometric technique that is described below. The U3Rs of the HECs obtained by the present invention are equal to or less than 0.21, preferably less than 0.18 while the HE-MS is in the range of 0.7 to 1.3.

Trimers, oligomers with a degree of polymerization (DP) of 3 anhydroglucose units, and other compounds of structure 1 are made by partial methanolysis of permethylated HEC derivatives. It is assumed that the cleavage of the permethylated HEC-backbone is a random process and that the formed oligomers of structure 1 have an EG-distribution that is representative for the EO-distribution of the whole sample.

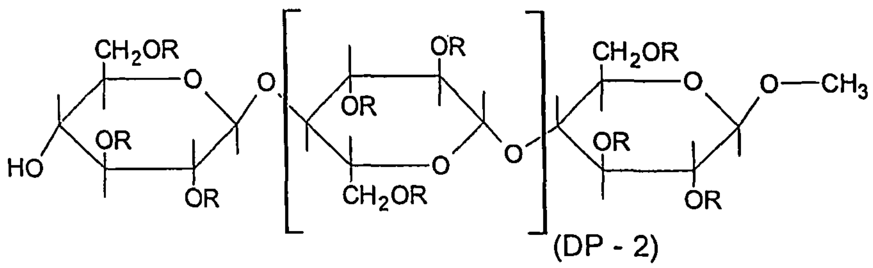

$R = (C_2H_4O)_n-CH_3$

Structure 1

In general, permethylated derivatives of HEC polymers can be prepared by the methylation reaction that is applied in the methylation analysis procedure for polysaccharides. (See publications of F.-G. Hanisch, Biological Mass Spectrometry, 23 (1994) 309-312; B. Lindberg, U. Lindquist and O. Stenberg, Carbohydrate Research, 170 (1987) 207-214; and P.W. Arisz, J.A. Lomax, and J.J. Boon, Carbohydrate Research, 243 (1993) 99-114.)

**Unsubstituted Trimer Ratio (U3R) Determination**

More specifically, in the present invention, the investigated HEC polymers are dissolved or swollen in dimethyl sulphoxide (DMSO). The hydroxyl groups in the polymer are deprotonated using a lithium methylsulphinyl carbanion solution in DMSO and they are converted to methoxyl groups by the reaction with methyl iodide.

The obtained permethylated HEC polymer is purified. More specifically, the permethylated HEC polymer is extracted in three extraction steps with chloroform from an aqueous DMSO lawyer that is acidified to pH < 2 with hydrochloric acid. The pooled chloroform extracts are washed four times with water. Some methanol is added after the last wash step and all solvents are evaporated.

The permethylated polymer is partially degraded by methanolysis. More specifically, the permethylated polymer is dissolved / swollen in methanol. Sufficient hydrochloric acid in methanol is added to get a hydrochloric acid concentration of about 0.50 molar. The sample is dissolved completely at 50°C for 15 minutes. Partial methanolysis is done at 70°C for 2.5 hours. The reaction is quenched by the addition of 2-methyl-2-propanol and all solvents are evaporated, yielding a residue that is composed of a mixture of oligomers of structure 1.

The residue is dissolved in methanol and a fraction of this sample is mixed with 2,5-dihydroxybenzoic acid solution that is spiked with sodium iodide. Mass spectra of the oligomer mixture are recorded with a Bruker Reflex II MALDI-TOF-MS (matrix assisted laser desorption ionization - time of flight - mass spectrometer), which instrument is equipped with a microchannel plate detector. The compounds of structure 1 are measured as their sodium ion adducts. The mass numbers of the monoisotopic mass peaks of the trimers are m/z 667.32, 711.34, 755.35, 799.39, etc. It is assumed that all trimers are measured with equal probability, independent of their molar HE-substitution, chain length of the substituents and their positions in the anhydroglucose residues.

Trimer fractions are derived by two data processing steps from the measured peak intensities of their monoisotopic mass peaks. First the background signal of the MALDI spectrum is subtracted from the measured peak intensities. Secondly, mainly due to $^{13}C$-isotopes that are incorporated in structure 1 the monoisotopic mass peaks make up only 70.6, 68.9, 67.2, 65.6 %, etc of all isotopes of trimers having 0, 1, 2, 3, etc attached EO-units, respectively. Unfortunately, the peak intensities of $^{13}C$-isotopes can not be measured accurately by MALDI-TOF-MS because of the recovery time that is needed for the microchannel plate detector after an intense mass peak has been recorded. In order to compensate the signal for the missing contribution of $^{13}C$-isotope peaks, the background corrected monoisotopic mass peak intensities are multiplied by a correction factor that is calculated from the theoretical isotope composition of the trimers. This factor increases with increasing number of C-atoms in structure 1, and values have been used of 1.417, 1.452, 1.488, 1.525, etc for trimers having 0, 1, 2, 3, etc attached EO-units, respectively.

Figure 1 shows an example of the EO-distribution profile of trimers that are derived from a HEC polymer. The fraction of unsubstituted trimers is indicated in gray. The most abundant class of trimers in this example is that of trimers with 7 attached EO-units. This class is indicated in white. The unsubstituted trimer ratio, i.e. the gray fraction divided by the white fraction, is calculated to be 0.121 for this example. It should be noted that the number of EO-units in the most abundant class of trimers varies, depending on factors as the molar substitution of the HEC and the process type by which the HEC was made, for example.

HEC derivatives that contain secondary substituents such as nonionic, cationic, and anionic substituents and mixtures thereof are analyzed similarly as non-modified HECs. In the case of modification levels smaller than 3.5 substituents per 100 monomer units, such as associative hydrophobic reagents for example, less than 10 % of the trimers are modified and consequently the fraction of modified trimers can be neglected.

The fraction of unmodified trimers decreases with increasing degree of substitution (DS) of the modifying agent. If the secondary substituent distribution is random along the cellulose backbone, than only half of the trimers would remain unmodified at a DS level of 0.21. The carboxymethyl (CM)-modified HEC listed in Table 2 has a CM-DS value in this order of magnitude and it is concluded for this sample that the fraction of CM-modified trimers cannot be neglected.

Furthermore, CM-groups that are attached to the HEC-backbone are converted into their methylesters by the derivatization procedure. The sodium ion adduct of dimers with two attached EO-units and two attached CM-groups has *m/z* 667.28. The mass resolution of MALDI-TOF-MS is insufficient to separate this mass peak from m/z 667.32, i.e. the mass peak of unsubstituted trimers, so that an accurate measurement of the U3R for carboxymethylated HEC-derivatives is not applicable (N/A).

**Water Solubility Determination:**

A 0.25 or 0.50 wt % HEC solution in water is prepared and mixed for at least 2 hours until the polymer is dissolved. The solution is filtered through a 10 - 15 micron fritted filter. The filtrate is dried in a vacuum oven at 102° C for at least 24 hours or until there is no weight change over two hours. The percent soluble is determined by gravimetry as:

$$\left(1-\frac{\text{Weight polymer recovered from filtered solution}}{\text{Weight polymer in unfiltered solution}}\right)\times 100$$

**APPLICATIONS:**

Many of these HEC samples exhibit novel and highly desirable rheology and performance properties in end use systems. In accordance with the present invention, the viscosity builds up not only by means conventional to HEC, but also is boosted significantly by molecular association. The association becomes stronger in low water activity systems and leads to network formation manifesting as gel-like properties in lean solvents and aqueous based functional systems. The HECs and derivatives made in the present invention have been shown to lower the HEC use-level needed and to provide rheology attributes that are unique as compared to other prior art HECs.

Furthermore, these HECs and derivatives thereof may be used in applications where there is a need for a specific rheology characteristic, e.g., viscosity, thixotropy, yield stress, elasticity, or solid state characteristics such as thermoplasticity and film flexibility. Examples of functional systems include aqueous based coatings (e.g., latex paints),

building and construction materials (e.g., cements, plasters), personal care products (e.g., skin care, hair care, oral care, nail care, and personal hygiene products), household care products (e.g., industrial cleaning liquids, pet care products), pharmaceuticals (e.g., excipients for tablets, capsules, and granules), oilfield applications (e.g., drilling fluids, completion fluids, and fracturing fluids), civil engineering, printing inks, adhesives, paper coating formulations, and retention and drainage aids In paper making.

The functional system can either be prepared in a continuous or batch process and either in a stepwise addition of the ingredients or a simple mixing of all of the ingredients at once. The order of addition of the ingredients can also vary over a wide range of additions. For example, the functional ingredients can be individually added one at a time to the formulation or all at once or the low HE-MS, water soluble HEC products can be added directly to the formulated ingredients in a single step. Hence, the process of thickening an aqueous based functional system (e.g., personal care products, household care products, oil field servicing fluids, civil engineering servicing fluids, paper coating products, paper making compositions, building and construction fluids, ceramic glazes, mineral processing products, and water based protective coatings such as architectural and industrial coatings), includes adding and mixing a sufficient amount of the low HE-MS, water soluble HEC polymer of the present invention that is compatible with the aqueous based functional system to thicken the functional system. The resulting functional system has comparable or better rheology and viscosity properties as compared to when using similar thickening agents including commercial HECs.

## PERSONAL CARE

When the composition is a personal care composition, it includes (a) from 0.1 % to 99.0 % by weight of the vehicle component and (b) at least one active personal care ingredient.

The personal care active ingredient must provide some benefit to the user's body. Personal care products include hair care, skin care, oral care, nail care, and personal hygiene products. Examples of substances that may suitably be included in the personal care products as active ingredients invention are as follows:

1) Perfumes, which give rise to an olfactory response in the form of a fragrance and deodorant perfumes which in addition to providing a fragrance response can also reduce body malodor,
2) Skin coolants, such as menthol, methyl acetate, methyl pyrrolidone carboxylate N-ethyl-*p*-menthane-3-carboxamide and other derivatives of menthol, which give rise to a tactile response in the form of a cooling sensation on the skin;
3) Emollients, such as isopropyl myristate, silicone oils, mineral oils and vegetable oils which give rise to a tactile response in the form of an increase in skin lubricity;
4) Deodorants other than perfumes, whose function is to reduce the level of or eliminate micro flora at the skin surface, especially those responsible for the development of body malodor. Precursors of deodorants other than perfume can also be used;
5) Antiperspirant actives, whose function is to reduce or eliminate the appearance of perspiration at the skin surface;
6)Moisturizing agents that keep the skin moist by either adding moisture or preventing moisture from evaporating from the skin;
7) Cleansing agents that remove dirt and oil from the skin;
8) Sunscreen active ingredients that protect the skin and hair from UV and other harmful light rays from the sun. In accordance with this invention a therapeutical effective amount will normally be from 0.01 to 10% by weight, preferably 0.1 to 5% by weight of the composition;
9) Hair treatment agents that condition the hair, cleans the hair, detangles hair, act as styling agents, volumizing and gloss agents, ahti-dandruff agent, hair growth promoters, hair dyes and pigments, hair perfumes, hair relaxer, hair bleaching agent, hair moisturizer, hair oil treatment agent, and anti-frizzing agent;
10) Shaving products, such as creams, gels and lotions and razor blade lubricating strips;
11) Tissue paper products, such as moisturizing or cleansing tissues;
12) Beauty aids, such as foundation powders, lipsticks, and eye care; and
13) Textile products, such as moisturizing or cleansing wipes.

In personal care compositions, the rheology modifiers obtained in the present invention can be used either alone or may also be used in combination with other known rheology modifiers including, but not limited to, polysaccharides (e.g., carrageenan, pectin, alginate), cellulose ethers, biopolymers (e.g., xanthan gum), synthetic polymers, and abrasive/thickening silicas.

## HOUSEHOLD CARE

When the composition is a household care composition, it includes (a) from 0.1 % to 99.0 % by weight of the

vehicle component and (b) at least one active household care ingredient.

The household care active ingredient must provide some benefit to the user. Household care products include fabric care, laundry detergent, hard surface cleaner, industrial institutional liquid soaps, and dish detergents. Examples of active ingredients or substances that may suitably be included according to the present invention are as follows:

1) Perfumes, that give rise to an olfactory response in the form of a fragrance and deodorant perfumes that in addition to providing a fragrance response can also reduce odor;
2) Insect repellent agent whose function is to keep insects from a particular area or attacking skin;
3) Bubble generating agent, such as surfactants which generates foam or lather;
4) Pet deodorizer such as pyrethrins that reduce pet odor;
5) Pet shampoo agents and actives, whose function is to remove dirt, foreign material and germs from the skin and hair surfaces;
6) Industrial grade bar, shower gel, and liquid soap actives that remove germs, dirt, grease and oil from skin, sanitize skin, and condition the skin;
7) All purpose cleaning agents that remove dirt, oil, grease, and germs from the surfaces in areas such as kitchens, bathroom, and public facilities;
8) Disinfecting ingredients that kill or prevent growth of germs in a house or public facility;
9) Rug and Upholstery cleaning actives that lift and remove dirt and foreign particles from the surfaces and also deliver softening and perfumes;
10) Laundry softener actives that reduce static and makes fabric feel softer;
11) Laundry detergent ingredients that remove dirt, oil, grease, and stains and kill germs;
12) Dishwashing detergents that remove stains, food, germs;
13) Toilet bowl cleaning agents that remove stains, kill germs, and deodorize;
14) Laundry prespotter actives that help in removing stains from clothes;
15) Fabric sizing agents that enhance appearance of the fabric;
16) Vehicle cleaning actives that remove dirt, grease, etc. from vehicles and equipment;
17) Lubricating agents that reduce friction between parts; and
18) Textile products, such as dusting or disinfecting wipes.

In household care compositions, the rheology modifiers obtained by the present invention can be used either alone or may also be used in combination with other known rheology modifiers including, but not limited to, polysaccharides (e.g., carrageenan, pectin, alginate), cellulose ethers, biopolymers (e.g., xanthan gum), synthetic polymers, and abrasive/thickening silicas.

The above are only limited examples of personal care and household active ingredients and are not a complete list of active ingredients' that can be used. Other ingredients that are used in these types of products are well known in the industry. In addition to the above ingredients conventionally used, the composition can optionally also include ingredients such as colorants, preservatives, antioxidants, nutritional supplements, activity enhancers, emulsifiers, viscosifying agents (such as salts, e.g., sodium chloride, ammonium chloride and potassium chloride), water-soluble polymers (e.g., HEC, modified HEC, carboxymethylcellulose), and fatty alcahols (e.g., cetyl alcohol), alcohols having 1-6 carbons, and fats and oils.

## PROTECTIVE COATINGS

Water-based protective coating compositions (commonly referred to as paints) in which cellulose ether derivatives are commonly used include latex paints or dispersion paints, of which the principal ingredient is the film-forming binders that include latices such as styrene-butadiene copolymers, vinyl acetate homopolymers and copolymers, and acrylic homopolymers and copolymers. Other binders that are typically used in paints include alkyd resins, and epoxy resins. Typically, they also contain opacifying pigments, dispersing agents and water-soluble protective colloids, the proportions being, by weight of the total composition, 10 parts to 50 parts of a latex, 10 parts to 50 parts of an opacifying pigment, 0.1 part to 2 parts of a dispersing agent, and 0.1 part to 2 parts of a water-soluble protective colloid.

These protective coatings can be either aqueous based architectural or industrial coating compositions. Architectural coatings are intended for on-site application to interior or exterior surfaces of residential, commercial, institutional or industrial buildings. Industrial coatings are applied to factory-made articles before or after fabrication, usually with the aid of special techniques for application and drying.

Water-soluble polymers conventionally used in the manufacture of latex paints include casein, methyl cellulose, hydroxyethylcellulose (HEC), sodium carboxymethyl cellulose (CMC), polyvinyl alcohol, starch, and sodium polyacrylate. The HECs of the present invention can be used as rheology modifiers for water-based protective coating compositions.

## PAPER COATINGS AND PAPER MAKING

Paper coating is a process in which the surface structure of paper or board is improved by applying a mineral coating that is subsequently dried. Coating process is the application of a water-borne pigment slurry, which is bound at the surface by one of several binders. Other coating components can be added to obtain a suitable rheology, and to impart properties such as brightness or water resistance.

A coating process can generally be divided into three different phases: (1) preparation of the coating formulation (known as called coating color), (2) coating and (3) drying. The general principles of formulating paper coating are mostly well known. Moreover, each paper maker has his own tailor-made recipes for his specific requirements. Therefore, it would not be possible to give a "recipe" for a specific coating process, coating type or printing process. However, a generic coating formulation recipe contains 75 - 90 % pigment (such as clay, satin white, calcium carbonate, titanium dioxide, talc, aluminum hydroxide, calcium sulfate, barium sulfate, synthetics, etc.), 0.10 - 0.50 % dispersant, 0.05 - 0.30 % alkali, 5 - 20 % binders (such as styrene-butadiene latices, acrylics, polyvinyl acetate, starch and starch derivatives, proteins such as casein, soya) and 0 - 2 % co-binder (cellulose ethers, polyvinyl alcohol and solution or polyacrylates emulsion). Other functional additives such as lubricants, optical brightening agents and defoamers are often added to the coating formulation. All amounts of ingredients are based on weight of pigment. The HECs of the present invention can be used as rheology modifiers for water-borne paper coating compositions.

In addition to paper coating, the HECs obtained by the present invention can be used in papermaking process and for surface sizing. In papermaking process, the low HE-MS, water soluble HEC can be used as an additive in the stock as a refining agent, wet-strength agent, dry strength agent, internal bonding agent, water retention agent and improving the sheet formation. For surface sizing, the low HE-MS, water soluble HEC can be used as a binding agent and aiding in film formation.

## OILFIELD SERVICING FLUIDS

Drilling an oil or gas well is a complex operation, involving several steps before and after the well is put into production. Primary oil-recovery operations include drilling the well, cementing the casing to the formation and completing the well prior to oil or gas production. Workover operations may be necessary during remedial work in producing wells, usually as an attempt to enhance or prolong the economic life of the well. When the flow rate of the fluid is diminished, the reservoir may be treated in some manner to increase the flow of fluid into the wellbore. This operation is called secondary recovery, known as fracturing/stimulation operations. They are performed either by acid wash or hydraulic fracturing. When the reservoir is depleted, enhanced oil recovery operations may be needed to increase the production rate. This operation is called tertiary recovery, and involves injection of fluids into the formation surrounding the production well to increase the flow rate of the formation fluid into the wellbore.

Drilling fluids are an integral clement of the drilling program for primary oil recovery. They are especially designed to perform numerous functions that condition the success of drilling operations. Their principal functions include, but not limited to, are:

- An effective hole cleaning efficiency (H.C.E.).
- Maintaining the stability of the open hole-formation.
- Formation of a thin and low-permeability filter cake on the formation.
- Minimizing formation damage.
- Friction reduction between the drilling string and the formation.
- Cool and clean the drill bit.

To perform these functions, drilling fluids should possess particular properties with regard to rheology, density, and filtration control. Filtration control is a key performance attribute that affects all other properties. In fact, loss of significant amount of water from the drilling fluid into the formation would result in irreversible change of the overall drilling fluid properties (density and rheology) that would seriously affect the stability of the borehole.

Among a variety of additives, carboxymethyl cellulose (CMC), HEC and polyanionic cellulose (PAC) are widely used to optimize water-based drilling fluid properties. High-viscosity types are used for rheology and fluid loss control properties while low viscosity types are exclusively used for filtration control properties. In most cases, these types are used together in a drilling fluid composition. During drilling operations, optimum drilling fluid attributes are further achieved by combining different components including clay, CMC/PAC, xanthan gum (primary rheology modifier), starches (improved filtration control) and other synthetics polymers that may be required for dispersing or shale inhibition properties.

Completion and workover fluids are specialized fluids used during well completion operations and remedial workover procedures. They are placed across the chosen pay zone after the well has been drilled but prior to putting it on production. These fluids must control not only subsurface pressure with density, but also must minimize formation

damage during completion and workover operations to improve oil or gas production rate. Because all wells are susceptible to formation damage to some degree (from a slight reduction in the production rate to complete plugging of specific zones) and the potential for permanent damage is greater during completion and workover operations than it is during drilling, It Is imperative to use a fluid that causes the least possible damage to the pay zone formation. The principal functions of completion and workover fluids include, but not limited to, are:

- Control subsurface pressures.
- Minimize formation damage.
- Maintain Well bore stability.
- Control fluid losses to the formation.
- Transport solids.
- Maintain stable fluid properties.

The types of completion and workover fluids can be categorized into clear solids-free brines, polymer viscosified brines with bridging/weighting agents, and other fluids including oil base, water base, converted muds, foam, etc. The primary selection criteria for an appropriate completion or workover fluid are density. Clear, solids free brines are the most commonly used fluids and are viscosified with polymers (CMC/PAC, xanthan gum, guar and guar derivatives, and HEC) and may incorporate solids that can be dissolved later, such as acid soluble calcium carbonate or sized sodium chloride salt, for increased density or bridging purposes. While HEC is the most suitable polymer for brine based systems, CMC/PAC and xanthan gum find their use in low density (up to 1.44 kg/l (12 ppg)) monovalent salts based brines.

Hydraulic fracturing may be defined as the process in which fluid pressure is applied to the exposed reservoir rock until failure or fracturing occurs. After failure of the rock, a sustained application of fluid pressure extends the fracture outward from the point of failure. This may connect existing natural fractures as well as provide additional drainage area from the reservoir. The fluid used to transmit the hydraulic pressure to the reservoir rock is called the fracturing fluid. To prevent the fracture from closing when pumping is stopped, propping agents, such as sized sand, are added to the fracturing fluid. The propping agent acts as supports to hold the fracture open after the treatment and to provide an improved ability of the fracture to conduct oil or gas through the fracture to the wellbore.

The low HE-MS, water soluble HECs and derivatives thereof obtained in the present invention can be used as Theology modifiers for aqueous based oilfield servicing fluids with improved efficiency.

## CIVIL ENGINEERING SERVICING FLUIDS

Civil engineering applications include tunneling, diaphragm walling, pilling, trenching, horizontal drilling, and water-well drilling. These applications are often characterized by their closeness to agglomerations where strict environmental regulation is in effect to minimize any kind of pollution or contamination. The corresponding working sites are further characterized by the availability of very poor mixing equipment on-site to efficiently disperse and dissolve the water-soluble polymers (WSPs). There is a desire in civil engineering applications for polymer suspensions that are stable, environmentally friendly, and meet all discharge regulations.

The low HE-MS, water soluble HEC and derivatives thereof obtained in the present invention are used as rheology modifiers in fluids for civil engineering applications including tunneling, piling, diaphragm walling, drilling, and bentonite doping.

## CONSTRUCTION / BUILDING COMPOSITIONS

Building compositions, also known as construction materials, include concrete, tile cement and adhesives, projection plasters, stuccos based on cement and synthetic binders, ready mixed mortars, manually applied mortars, underwater concrete, joint cement, joint compounds, gypsum board, crack fillers, floor screeds, and adhesive mortars. These compositions are essentially Portland cements, Plaster of Paris or vinyl copolymers containing functional additives to impart characteristics required for various construction applications.

The joint cement can contain clay and mica or can be clay free (i.e., contain less than 0.5 wt % clay). While lime was once the preferred material for controlling the water ratio in the building compositions, cellulose ethers are at present time the most used because of their contribution to improve the water retention characteristics and other physical properties such as workability, consistency, open time, tack, bleeding, adhesion, set time, and air entrainment.

The low HE-MS, water soluble HEC and derivatives thereof obtained in the present invention are used as rheology modifiers in the above mentioned construction and building material compositions.

## PHARMACEUTICALS

Pharmaceutical compositions normally are in the form of tablets, capsules, or granules. The sole purpose of a pharmaceutical composition, regardless of its form, is to deliver a therapeutically active medicament to the desired place of use. The most common form of the medicament delivery system is the tablet form. In the tablet or capsule form, it is common practice to use at least one inert ingredient for production, delivery, and economic considerations. Examples of inert ingredients are excipients, diluents, fillers, and binders. The combination of the medicament with the inert ingredients provides a formulation that can be directly compressed into tablets or made into granules or agglomerations for encapsulation. In order to provide a directly compressible product, these excipients must have certain physical properties, including flow ability, sufficient particle size distribution, binding ability, acceptable bulk and tap densities, and acceptable dissolution properties in order to release the medicament upon oral administration.

The tow HE-MS, water soluble HECs or derivatives thereof obtained in the present invention can be used as a pharmaceutical excipient in free flowing, directly compressible slow release granule compositions that can be prepared by dry-blending, roller-compaction, or wet-agglomeration. The pharmaceutical composition may contain from 5 to 80 % by weight of the low HE-MS, water soluble HEC or HEC derivative. The pharmaceutical composition can also contain an inert filler in the amount of from 0.01 to 95 % by weight. Examples of the pharmaceutical fillers are monosaccharides, disaccharides, polysaccharides, polyhydric alcohols, inorganic compounds, and mixtures thereof. This pharmaceutical composition can also contain from 0.01 to 50 % of an additional control release agent such as cellulose ethers, cellulose esters, polyethylene oxides, polyvinyl alcohol and copolymers, methacrylic acid derivatives, waxy-fatty materials, natural hydrocolloids, and Carbopol® derivatives.

A controlled release pharmaceutical tablet for oral administration is composed of from 5 to 80 % by weight of the total composition of the low HE-MS, water soluble HEC or derivatives there'of, up to 90 % by weight of an inert pharmaceutical filler (as mentioned above), and an effective amount of a therapeutically active medicament to render a therapeutic effect. The ratio of medicament to the low HE-MS, water soluble HEC (hydrophilic material) is based in part upon the elative solubility of the medicament and the desired rate of release. By varying this ratio and / or the total weight of the tablet, one can achieve different slow release profiles, and may extend the dissolution of some medicaments to 24 hours.

An immediate release tablet composition is composed of from 0.5 to 10 % by weight of the low HE-MS, water soluble HEC, suitable fillers and tableting aids, and an effective amount of a therapeutically active medicament The amount of the active medicament depends on the desired amount needed to deliver the desired effect.

## EXAMPLES

The following Examples indicate various cossible methods for making, describing, and using the HECs in accordance with the present invention. These Examples are merely illustrative, and are not to be construed as limiting the present invention to particular compounds, processes, conditions, or applications. All parts and percentages are by weight unless otherwise stated.

### PROCEDURES FOR PREPARING SAMPLES

Table 1 shows the details of the individual Examples. Cellulose, water, and solvent were charged to a nitrogen-sparged, reaction kettle per the ratios described in the various tables. The reactor was inerted with nitrogen. The caustic was added to reach the desired alkali to AGU molar ratio (AC1) and the alkali cellulose slurry temperature was maintained at 20° C for approximately 1 hour. Ethylene oxide was added to the reaction mixture. Acid was then added continuously over a 30 minute heat-up to 60° C and a 30 minute hold at 60° C in order to reach the desired alkali to AGU molar ratio (AC2). The temperature was raised to 100° C and held for 60 minutes to complete the hydroxyethylation. The reaction mixture was cooled down to ambient temperature and neutralized with sufficient acid to neutralize any excess alkali. The HEC product was then purified, dried, and ground to the desired particle size.

For modified HEC, after cooling the HEC reaction mixture to ambient temperatures, caustic was added to achieve the desired alkali/AGU molar ratio'for modification (ACM). The modifiying agents were charged. When the modification was hydrophobic, the reactor was heated to 115° C and the temperature was maintained for 2.5 hours. When the modification was anionic or cationic, the reactor was heated to 60° C and the temperature was maintained for 2.5 hours. Upon completing the modification reaction, the reaction mixture was cooled down to ambient temperature and neutralized with sufficient acid to neutralize any excess alkali. The product was then purified, dried, and ground to the desired particle size.

Table 1: Sample Preparation Descriptions

| EX | Cellulose Fumish$^A$ | Cell. Bone Dry (g) | IPA (g)$^B$ | Acid Type and Concentration | $H_2O$: AGU$^C$ | NaOH: AGU$^D$ AC1 | NaOH: AGU AC2 | NaOH: AGU ACM | EO (g) | HM$^E$ (g) | Callonic or Anionic Agent (g) |
|---|---|---|---|---|---|---|---|---|---|---|---|
| EX 1 | Ii | 58 | 667 | 45% acetic in IPA | 16.7 | 1.74 | 0.20 | - | 33.3 | - | - |
| EX 2 | Ii | 58 | 667 | 50% acetic in IPA | 16.7 | 1.74 | 0.08 | - | 31.7 | - | - |
| EX 3 | Ii | 58 | 667 | 50% acetic in IPA | 16.7 | 1.74 | 0.08 | 0.08 | 31.5 | Butyl GE$^F$ 5.8 | - |
| EX 4 | Ii | 58 | 667 | 50% acetic In IPA | 16.7 | 1.74 | 0.08 | 0.73 | 31.6 | - | SCA$^G$ 16 |
| EX 5 | Ii | 58 | 667 | 50% acetic in IPA | 16.7 | 1.74 | 0.08 | 0.73 | 31.9 | - | 60 % Quat 188$^H$ 5.94 |
| EX 6* | Ii | 58 | 667 | 50% acetic In IPA | 16.7 | 1.74 | 0.08 | - | 23.9 | - | - |
| EX 7 Paint Example | I | 58 | 667 | 50% acetic In IPA | 16.7 | 1.74 | 0.08 | - | 38.1 | - | - |
| EX 8 | I | 58 | 667 | 50% acetic in IPA | 16.7 | 1.74 | 0.08 | - | 31.6 | - | - |
| EX 9 | Iv | 58 | 667 | 50% acetic in IPA | 16.7 | 1.74 | 0.08 | - | 38.0 | - | - |
| EX 10 | Iii | 58 | 067 | 45% acetic in IPA | 16.7 | 1.74 | 0.20 | - | 35.0 | - | - |
| EX 11 | Iii | 58 | 667 | 50% acetic in IPA | 16.7 | 1.74 | 0.08 | - | 30.2 | - | - |
| EX 12 | I | 58 | 667 | 35% acetic In IPA | 16.7 | 1.74 | 0.40 | - | 42.2 | - | - |
| EX 13 | I | 58 | 667 | 45% acetic In IPA | 16,7 | 1.74 | 0.20 | - | 32.5 | - | - |
| EX 14 | I | 58 | 667 | 45% acetic In IPA | 16.7 | 1.74 | 0.20 | - | 38.8 | - | - |

(continued)

| EX | Cellulose Fumish[A] | Cell. Bone Dry (g) | IPA (g)[B] | Acid Type and Concentration | $H_2O$: AGU[C] | NaOH: AGU[D] AC1 | NaOH: AGU AC2 | NaOH: AGU ACM | EO (g) | HM[E] (g) | Callonic or Anionic Agent (g) |
|---|---|---|---|---|---|---|---|---|---|---|---|
| EX 15 | I | 58 | 667 | 50% acetic In IPA | 16.7 | 1.74 | 0.04 | - | 47.8 | - | - |

[A]I Cotton LInter, Intrinsic Viscosity I.V.(dl/g) > 20
II Wood Pulp, I.V.(dl/g) 4 - 8
III Wood Pulp. I.V.(dl/g) 11 - 15
IV Wood Pulp, I.V.(dl/g) 18
[B]The solvent weight Includes the solvent delivered to the reactor during the acid quench (total solvent). IPA=Isopropanol
[C]Molar ratio of water to anhydroglucose (AGU)
[D]Molar ratio of sodium hydroxide (NaOH) to anhydroglucose (AGU)
[E]Hydrophobe modification (HM)
[F]GE=Glycidal Ether
[G]SCA=Sodium MonoChloroacetate
[H]Quat 188=cationizing agent - N-(3-chloro-2-hydroxypropyl)trimethylammonium chloride
*Reference Example, not in accordance with the invention

**Examples 1 - 5 and Reference Example 6**

HECs that have an HE-MS between 0.7 and 1.3, high water solubility, and an unsubstituted trimer ratio less than 0.21 are the basis of this invention. The properties for HECs of Examples 1 - 5 of this invention are shown in Table 2. Table 1 describes how the lowly hydroxyethylated HECs are prepared by completely opening up the cellulose fiber with high initial caustic level (AC1) and then "quenching" continuously to a low caustic level (AC2) during the reaction. This process drives a more uniform substitution so as to render high water solubility at low HE-MS. Examples 1-5 in Table 2 have an unsubstituted trimer ratio (U3R) less than 0.12 indicative of a very uniform structure and water solubility well over 90%. Reference Example 6 describes an HEC with HE-MS of 0.7 having 100% water solubility, whereas the U3R is 0.26 setting the lower HE-MS limit of the invention.

Also shown in Table 2 are the properties of commercial Natrosol HECs with HE-MS in the range of 1.5 to 1.8. Commercial HECs with HE-MS in the range of 0.7 to 1.3 are not available due to poor water solubility and viscosifying power. For HECs of the invention, unprecedented water-solubility and unsubstituted trimer ratios are achieved at significantly lower HE-MS. Examples 3 - 5 demonstrate that further modification with hydrophobic, cationic, and anionic reagents is feasible.

**Table 2 - Water Soluble Low HE-MS HEC and Derivatives**

| Example | HE-MS | Derivative | 1% Aq. Viscosity (cP) (mPa·s) | Solubility | Unsubstituted Trimer Ratio U3R |
|---|---|---|---|---|---|
| Example 1 | 1.2 | - | 244 - 2% | 94 | 0.075 |
| Example 2 | 1.0 | - | 191 - 2% | 99 | 0.110 |
| Example 3 | 1.0 | Butyl 0.6% | 227-2% | 95 | 0.079 |
| Example 4 | 0.9 | Carboxymethyl DS 0.23 | 105-2% | 95 | N/A |
| Example 5 | 0.9 | Quat 1.5 % | 221-2% | 95 | 0.119 |
| Example 6* | 0.7 | - | 24,200 - 2% | 100 | 0.260 |
| Comparative Natrosol 150GXR | 1.5 | - | 200 - 2% | 94 | 0.191 |
| Comparative Natrosol 150GBXR | 1.6 | - | 185-2% | 97 | 0.031 |
| Comparative Natrosol 180GXR | 1.8 | - | 325-2% | 93 | 0.167 |
| Example 7 | 1.1 | - | 24,860 | 94 | 0.070 |
| Example 8 | 0.9 | - | 24,360 | 92 | 0.102 |
| Example 9 | 1.1 | - | 10,680 | 93 | 0.088 |
| Example 10 | 1.3 | - | 5,280 | 95 . | 0.099 |
| Example 11* | 0.9 | - | 4,860 | 90 | 0.142 |
| Example 12* | 1.3 | - | 10,100 | 98 | 0.161 |
| Example 13 | 1.1 | - | 27,200 | 95 | 0.102 |
| Example 14* | 1.0 | - | 32,100 | 90 | 0.180 |
| Example 15 | 0.8 | - | 29,600 | 95 | 0.119 |
| * Reference Example | | | | | |

**Examples 7 - 15**

Examples 7-15 demonstrate that the synthesis procedure can be performed on a wide range of cellulose furnishes from cotton linters to wood pulps in order to generate a family of water-soluble, low HE-MS HEC products.

Examples 7,8, and 12-15 show that high molecular weight, low HE-MS, water soluble HECs produced from

cotton linters have 1 wt % Brookfield viscosities (spindle 3, 3 rpm, at 25° C) up to 32,000 mPa·s (cps). Commercially available high molecular weight HECs such as those marketed under the trademarks Natrosol 250 HHBR & HHR, Natrosol HI-VIS, Cellosize QP 100 MH, and Tylose H 200000 YP2 products typically have 1 wt% viscosities in the range of 4,500 - 6,000 (mPa·s) (cP).

**Example 16**

Architectural Coatings

Water-soluble, low HE-MS HEC shows enhanced thickening efficiency in architectural coating applications. Example 7 and Natrosol 250HHR were evaluated in the Ucar Latex 379G 70-PVC flat paint formulation shown. The novel thickener was 30% more efficient than 250HHR while maintaining similar paint properties as shown in Table 3.

Ucar Latex 379G **70-PVC** Formulation

| **Base Paint** | **Grams/13,000g** |
|---|---|
| **Pigment Grind** | |
| Water | 2,521 g |
| Nuosept 95 | 32.4 |
| Tamol 731A Dispersant | 64.7 |
| Igepal CO-660 | 31.0 |
| Igepal CO-897 | 43.6 |
| AMP-95 | 14.1 |
| Propylene Glycol | 182.9 |
| Rhodine 640 | 14.1 |
| Water, Discretionary, | 1,407 |
| Ti-Pure R-931 TiO2 | 1,055 |
| ASP NC Clay | 2,814 |
| ECC #10 White Calcium Carbonate | 2,110 |
| Celite 281 Silica | 352 |
| -Disperse to Hegman 4 to 5 - | |
| **Letdown** | |
| -All Discretionary Water in - | |
| Ucar Latex 379G | 2,079 |
| Texanol | 11.1 |
| PA-454 Antifoam | 26.7 |
| Propylene Glycol | 140.7 |
| Total | 13,000 g |

**Thickened Paints**

| | |
|---|---|
| Base Paint | 220 g |
| Thickener Solution + Water to 95 KU | 50 |
| Total | 270 g |
| pH, initial (8.5 Target) | |
| Density, kg/100 l (lb/100 gal) | 1.35 (11.3) |
| Solids, Weight % | 47.8 |
| Volume% | 29.3 |
| PVC, %69.9 | |

**Table 3: Ucar Latex 379G 70-PVC Paint Properties**

| Thickener | Thickener Conc. (wt%) | Stormer (KU) Initial/ Overnight | HSV (Poise) (0.1 Pa·s) | Sag | Leveling | Hiding |
|---|---|---|---|---|---|---|
| Natrosol 250HHR | 0.43 | 100/100 | 0.6 | 8 | 5 | 0.984 |
| Example 7 | 0.33 | 100/100 | 0.6 | 8 | 4 | 0.983 |

**Example 17**

Completion / Workover Fluids

The HECs of the invention exhibits novel thickening of heavy brines. Completion fluids are composed of a variety of brines of different salinity characterized by a density ranging from 1.0kg/l (8.5 ppg (pound per gallon)) for seawater up to 2.3kg/l (19.2 ppg) for heavy brines containing zinc and calcium bromide salts. Standard high viscosity HEC is commonly used as a viscosifier for brines ranging from 1.1-1.6 kg/l (9 - 13 ppg). There presently is not an efficient viscosifier for heavy brines with a density ranging from 1.7kg/l (14 ppg) ($CaBr_2$) to 2.3 kg/l (19.2 ppg) ($ZnBr_2/CaBr_2$). These brines have a very low level of free water content available, and therefore, do not promote optimum hydration of standard HECs. These brines are characterized by a very low pH (pH< 1 for $ZnBr_2/CaBr_2$).

Invention HEC Examples 13 and 15 were evaluated in 4 different brine systems (freshwater, salt-saturated water, $CaBr_2$ and $ZnBr_2/CaBr_2$) at 0.57 wt %. These were compared to a standard HEC widely used in completion fluids (Natrosol HI-VIS). The viscosity and fluid loss properties were measured after static aging overnight at room temperature. Detailed results are reported in Tables 4-a to 4-d.

Invention Examples 13 and 15 showed exceptional thickening in the high density, heavy brine solutions (characterized by low water activity) as detailed by the high apparent viscosities (A.V.) and yield values (Yv) that developed in these systems (Tables 4 c-d). In contrast, commercial HI-VIS did not go into solution in these low water activity systems. Additionally, the invention HEC examples developed appreciable low-end rheology as reflected by the 6 and 3- rpm Fann dial readings, and showed appropriate fluid loss values.

Among the water soluble, low HE-MS HEC samples, Example 15 provided outstanding thickening efficiency in all systems (fresh water, seawater, and high-density brines). Solutions made with this invention example were so viscous in the heavy brines that the viscosity reading was out of scale suggesting an extremely versatile and efficient viscosifier in a variety of solutions.

| Table 4-a: Rheology/Fluid Loss performance of various HEC samples In Demineralized water | | | | | | | | | | |
|---|---|---|---|---|---|---|---|---|---|---|
| Fluid System | | | Sample | Final | Fann DR | | Rheology | | | Fluid Loss (ml) |
| Brine | Density (kg/l) | Initial pH | Ref. | Ph Aft. Ag. | 6 rpm | 3 rpm | A.V.(cPs) mPa·s | P.V.(cPs) mPa·s | Yv (*) (Lb/100ft2) | |
| Water | 1.0 (8.3 ppg) | 7.3 | Invention HEC Example 13 | 11.1 | 11 | 7 | 51 | 22 | 288 (59) | 45 |
| | | | Invention HEC Example 15 | 10.8 | 20 | 13 | 63 | 24 | 381 (78) | 90 |
| | | | Natrosol HI-VIS | 10,1 | 12 | . 8 | 45 | 19 | 254 (52) | 31 |
| (*) = kg/100 $m^2$ | | | | | | | | | | |

**Table 4-b: Rheologyl Fluid Loss performance of various HEC samples in 36% NaCl Solution**

| Fluid System | | | Sample | Final | Fann DR | | Rheology | | | Fluid Loss (ml) |
|---|---|---|---|---|---|---|---|---|---|---|
| Brine | Density (kg/l) | Initial pH | Ref. | Ph Aft. Ag. | 6 rpm | 3 rpm | A.V.(cPs) mPa·s | P.V.(cPs) mPa·s | Yv (*) (Lb/100ft2) | |
| Saturated Salt (36% NaCl) | 1.2 (10.0ppg) | 8.1 | Invention HEC Example 13 | 10.5 | 2 | 1 | 21 | 16 | 49 (10) | 14 |
| | | | Invention HEC Example 15 | 10.3 | 10 | 6 | 58 | 27 | 312 (64) | 21 |
| | | | Natrosol HI-VIS | 10.2 | 11 | 7 | 55 | 26 | 283 (58) | 31 |

(*) = kg/100 $m^2$

**Table 4-c: Rheology/ Fluid Loss performance of various HEC samples In CaBr2 Brine**

| Fluid system | | | Sample | Final | Fann DR | | | Rheology | | |
|---|---|---|---|---|---|---|---|---|---|---|
| **Brine** | **Density** | **Initial pH** | **Ref.** | **PH Aft. Ag.** | **6 rpm** | **3 rpm** | **A.V. mPas(cPs)** | **P.V. mPas(cPs)** | **Yv (*) (lb/100ft2)** | **Fluid Loss (ml)** |
| CaBr2 | **1.7 kg/l**(14.5ppg) | 7.43 | Invention HEC Example 13 | 7.5 | 52 | 38 | 140 | 63 | 752 (154) | 28 |
| | | | Invention HEC Example 15 | 7.6 | 84 | 66 | Out of Scale | | | 16 |
| Did not fully go into solution | | | Natrosol HI-VIS | 7.7 | 7 | 4.4 | 49 | 32 | 161 (33) | Blowout |

(*) = kg/100 $m^2$

| Table 4-d: Rheology/ Fluid Loss performance of various HEC samples In ZnBr2/CaBr2 brine | | | | | | | | | | |
|---|---|---|---|---|---|---|---|---|---|---|
| | **Fluid system** | | **Sample** | **Final PH Aft. Ag,** | **Fann DR** | | **Rheology** | | | |
| **Brine** | **Density** | **Initial** pH | **Ref.** | | **6** rpm | **3** rpm | **A.V. cPs** | **P.V. cPs** | **Yv (*) (lb/100ft2)** | **Fluid Loss (ml)** |
| $ZnBr^2/CaBr^2$ | 2.3 kg/l (19.2ppg) | 0.79 | Invention HEC Example 13 | 1.4 | 104 | 85 | Out of Scale | | | 147 |
| | | | Invention HEC 15 | 1.2 | 130 | 108 | Out of Scale | | | 138 |
| | | | Natrosol HI-VIS | 1.2 | | | Did not go Into solution | | | |
| (*) = kg/100 $m^2$ | | | | | | | | | | |

**Example 18**

Oil-well Cementing

Water soluble, low HE-MS HEC shows exceptional fluid loss properties at high temperature and in the presence of high salt. Oil-well cementing is the process of mixing a slurry of cement, water, and additives and pumping it down through steel casing to critical points in the annular space between the wall of the well and the outside of the casing. Additives are incorporated into cement slurries to deliver various functional properties, such as set time control, friction reduction, slurry density modification, cement bonding and fluid loss control. Among these properties, fluid loss control is a critical concern, especially difficult to control under severe environment conditions such as high salt and high temperature. To prevent early dehydration of cement slurries and related problems, fluid loss control additives are added to help maintain the integrity of designed slurry properties.

A wide variety of fluid loss control additives are currently used in formulating cement slurries depending upon the oil well environment. For medium temperature operations, cellulosics such as carboxymethylhydroxyethylcellulose (CMHEC) and hydroxyethylcellulose (HEC) are the most commonly used fluid loss additives. Among cellulosics, low molecular weight ones are preferred to achieve a good balance between fluid loss control and rheological properties by allowing higher flexibility of the polymer dosage under different operating conditions. While these polymers provide acceptable fluid loss control under moderate operating conditions, they are unsatisfactory under high temperature and salt environments. Hence, there is a need for improved fluid loss control additives under a wide range of operating conditions.

It has been surprisingly discovered that low molecular weight HECs with low HE-MS and high solubility, significantly reduce the fluid loss in oil well cements. The HECs of the invention show outstanding temperature and salt tolerance over standard commercially available cement grade HEC (Natrosol 180 GXR, available from Aqualon Company). Furthermore, butyl modified low HE-MS, water soluble HEC Example 3 performs better than any other comparative samples.

Invention examples 2 and 3, and Natrosol 180 GXR were evaluated in the cement slurry formulation shown containing 0 and 18% salt (based on water content). The slurry density was around 1.89-1.94 kg/l (15.8-16.2 ppg). Fluid Loss performance was measured at moderate and high temperatures, 27°C (80 °F) and 82°C (180 °F), respectively.

Formulation Cement Slurry Composition

| Ingredients | Type | Weight (g) |
|---|---|---|
| Cement | Dyckerhoff Red label | 600 |
| Dispersant | Lomar D | 3.3 |
| Fluid Loss Additive | HEC | 2.7 |
| Water | Distilled | 264 |

(continued)

| Ingredients | Type | Weight (g) |
|---|---|---|
| Salt | NaCl | 0<br>47.5 (18% basis weight on water) |
| Retarder | Calcium Lignosulfonate | 0 (27°C) (80 °F)<br>1.2 (82°C) (180°F) |

Table 5 demonstrates the superior performance of Invention Examples 2 and 3. The fluid loss control provided by the invention examples is less affected by temperature and salt than standard HEC. The invention examples show outstanding performance at 82°C (180 °F) with 18% salt. Under these extreme conditions, the fluid loss performance of Examples 2 and 3 are 10- 20 times better than commercial HEC.

Table 5: American Petroleum Institute (API) Fluid Loss Control Properties

| Sample | MS | C-4 modification | Fluid Loss @ 27°C (80 °F) | | Fluid Loss @ 82°C (180 °F) | |
|---|---|---|---|---|---|---|
| | | | w/o NaCl | with 18% NaCl | w/o NaCl | With 18% NaCl |
| HEC 180GXR | 1.8 | 0 | 30 ml | 224 ml | 54 ml | 766 ml |
| Invention Example 2 | 1.0 | 0 | 24 ml | 144 ml | 49 ml | 62 ml |
| Invention Example 3 | 1.0 | 0.56% | 23 ml | 127 ml | 46 ml | 37 ml |

**Example 19**

Paper

Low HE-MS, water solube HEC is a highly efficient thickener and water retention agent in paper coatings. Invention HEC Examples 1 and 2, commercial samples Aqualon 7L1T CMC, and Natrosol 250GR were evaluated as thickeners and water retention aids in the paper coating formulation as shown below. The amount of rheology modifier necessary to maintain the Brookfield viscosity at 1500 +/-50 mPa·s (cPs), the water loss, and Hercutes high shear viscosity are shown in Table 6. Low HE-MS, water soluble HECs Examples 1 and 2 and HEC 250GR are of similar molecular weights and solution viscosities; however, the low HE-MS, water soluble HEC products have a significantly higher dosage efficiency than HEC 250GR (up to 50% more efficient) while maintaining its low water loss rate. In addition Examples 1 and 2 have much lower water loss and higher dosage efficiency than Aqualon 7L1T CMC.

Paper coating formulation

| | Parts |
|---|---|
| HC 60* | 60 |
| HC 90* | 40 |
| Dow 620 SB latex | 12 |
| Calcium stearate | 1.00 |
| Dispex N-40 (dispersinq agent) | 0.25 |
| Solids (%) | 68+/-0.5 |
| Viscosity (mPa·s) (cps): | 1500 mPa·s (1500 cps) at ambient |
| Rheology modifier | Aqualon CMC7L1T, HEC 250GR, Invention,Examples 1 and 2 |
| * Ground Calcium Carbonate (HydroCarb) from OMYA Inc. | |

**Table 6: Paper Coating Properties**

| Thickener | HE-MS or DS | 2% Viscosity (cPs) (mPa·s) | Parts thickener/ 100 part filler | Water Loss (g/sq meter, 0.6 Bar/1min) | High Shear Rheology 1st Pass 2400/4400 RPM | High Shear Rheology 2nd Pass 2400/4400 RPM |
|---|---|---|---|---|---|---|
| Natrosol HEC 250G | 2.5 | 300 | 0.75 | 91 | 84/56 | 54/48 |
| Aqualon 7L1T CMC | 0.7 | 100 | 0.99 | 168 | 42/34 | 34/30 |
| Invention Example 1 | 1.2 | 244 | 0.50 | 96 | 66/50 | 55/46 |
| Invention Example 2 | 1.0 | 191 | 0.55 | 101 | 66/51 | 52/45 |

**Example 20**

Construction

Low HE-MS, water soluble HEC shows enhanced viscosity in joint compounds. Example 9 and Natrosol 250HHR product were evaluated as thickeners at 0.30 wt % in an all-purpose joint compound formulation, as described below. Table 7 shows that the formulation containing invention example HEC was more efficient (higher joint compound viscosity) while maintaining good adhesion, workability, and cratering properties.

All-Purpose Joint Compound Formulation

| Ingredients | Supplier | Wt% |
|---|---|---|
| Ground CaCO3 | Georgia White #9 | 61 |
| Attapulgite Clay | Gel B, Milwhite | 2.0 |
| Mica | 4-K, Oglebay Norton | 3.00 |
| Latex dispersion | EVA or PVA latex (see Note 1) | 2.5 |
| Propylene glycol | Aldrich | 0.35 |
| Biocide | Trosan 174, Troy chemical | 0.05 |
| Defoamer | Foamaster PD1WD, Cognis | 0.02 |
| Thickener | | 0.30 |
| Water | Tap water | 30.6 |
| Total | | 100 |

**Table 7: All Purpose Joint Compound Properties**

| Thickener | Joint Compound Viscosity (Brabender Units) | Adhesion | Cratering (1-10) 10 best |
|---|---|---|---|
| 250HHXR Commercial HEC | 480 | 100% | 7 |
| Invention Example 9 | 500 | 100% | 8 |

**Example 21**

Personal Care

Low HE-MS, water soluble HEC shows enhanced viscosity in personal care formulations. Natrosol® hydroxyethyl cellulose type 250HHR and low HE-MS, water soluble HEC Example 8 were compared at 0.7 wt% for thickening efficiency in the hair conditioner formulation shown below.

**Hair Conditioner**

| | |
|---|---|
| 90.94g | Deionized water |
| 00.70g | Thickening polymer (Natrosol® 250HHR, HEC example 2) |
| 02.00g | Cetyl alcohol |
| 00.50g | Potassium Chloride |
| 02.00g | Isopropyl Myristate |
| As required - | citric acid to adjust pH |
| As required - | Sodium hydroxide to adjust pH |
| 00.50g | Germaben II |

**Procedure:**

The thickening polymer was added to water under agitation. Next, the pH was adjusted to 8.0 to 8.5. The slurry was stirred for at least 30 minutes or until the polymer dissolved. The solution was heated to about 65°C and cetyl alcohol was added and mixed until homogeneous. The mixture was cooled to about 50°C and potassium chloride was added. Isopropyl myristate was added and mixed until the mixture looked homogeneous. The pH of the mixture was adjusted to 5.3 - 5.5 with citric acid and/or NaOH solution. The conditioner was preserved with 0.5 g Germaben II and mixed until the mixture reached room temperature.

The viscosity of the conditioning formulation containing low HE-MS, water soluble HEC Example 8 was 3,730 mPa·s (cP), as compared to the control containing Natrosol® 250HHR at 910 mPa·s (cPs), a 4-fold improvement.

**Example 22 (Reference Example)**

Oral Care - Lean Solvent Gels

Low HE-MS, water soluble HECs form strong gels in lean solvents unlike other commercial high molecular weight HEC. There is a need for efficient Viscosifiers and gelling agents in personal and oral care applications that function well in low water activity compositions such as hydro-alcoholic and lean solvent solutions. Lean solvents are the basis of toothpaste formulations and are typically a mixture of water and a miscible organic solvent such as sorbitol or glycerol. Often hydrophilic polymers have difficulty fully hydrating in these lean solutions similarly to solutions with high salt levels as there is less water accessible to the polymer.

It has been discovered that these water-soluble low HE-MS HEC samples act as efficient gelling agents in glycerol-water solutions, unlike commercial HECs. 1 wt% solutions of Reference Examples 12 and 14 and Natrosol 250 HHX were prepared in water. These three samples are of similar molecular weight. Upon diluting the low HE-MS samples with glycerol to 0.5 wt %, elastic gels developed over the course of a couple of days. Undisturbed samples of the gels did not exhibit significant syneresis over the course of 3 weeks. This behavior is unlike any of our standard Natrosol HEC materials that yield free-flowing solutions under both conditions.

Visual assessment of the 0.5 wt% solution quality and zero-shear viscosity for the three HECs tested are listed in Table 8. The distinctive behavior of the low HE-MS HEC samples is particularly evident when comparing zero shear viscosities in the 50:50 glycerol:water solutions. Reference Examples 12 and 14 reveal solution viscosities that are 6 - 10 times greater than expected. Furthermore, the rheological profiles are indicative of elastic gels with yield points.

The assembly is not instantaneous, but takes some time (hours-days) to form. This may find use in PC applications such as toothpaste formulations where the ability to easily pump a material into end-use packaging is desirable via in situ thickening after final packaging. Development of a yield stress is also advantageous in suspending solid ingredients. It is important to point out that all operations described here were performed without heating or a drastic change In solution pH. Competing technologies for the development of a yield stress, such as carrageenan (heating/cooling) or synthetic carbomer (pH control) employ those strategies, which can be incompatible with other ingredients in the

formulation.

In order to develop the gel-like character observed with these systems, the initial dissolution of the polymer in a strong solvent (i.e water) followed by dilution with a non-solvent as described above was followed. The use of 50:50 glycerol:water is one example of this. Replacement of the glycerol with any water miscible cosolvent (e.g. alcohols) should provide similar behavior.

**Table 8: Appearance and Zero Shear Viscosity of 0.5 wt% HECs in Water and in 50:50 Glycerol:Water Solutions**

| Sample | HE-MS | Solvent | Zero Shear Viscosity (Pa.s) | Solution Appearance @ 48 hours |
|---|---|---|---|---|
| Natrosol 250HHX | 2.4 | Water | 0.43 | Clear fluid |
| Example 12* | 1.3 | Water | 0.49 | Clear fluid |
| Example 14* | 1.0 | Water | 0.90 | Clear fluid |
| | | | | |
| Natrosol HHX | 2.4 | Glycerol:water | 3.2 | Clear fluid |
| Example 12* | 1.3 | Glycerol:water | 20.9 | Clear gel |
| Example 14* | 1.0 | Glycerol:water | 33.9 | Clear strong gel |
| * Reference Example | | | | |

**Example 23**

Household

Low HE-MS, water soluble HEC shows excellent thickening ability in a common household care formulation. Natrosol® hydroxyethyl cellulose type 250HHR CS and low HE-MS, water soluble HEC Example 8 were evaluated at 0.2 wt% for their compatibility, thickening efficiency, and clarity in an all-purpose cleaner. Table 9 shows that example 8 thickens the all-purpose cleaner nearly 2.5 times better than Natrosol® 250HHR CS which is of similar molecular weight. Furthermore, the clarity remains high indicating compatibility with this household cleaner.

**Procedure:**

20 g of a 1 wt% polymer solution was added to 80 g of Lysol All-Purpose Cleaner (pH 8). Final product viscosity was measured using the Brookfield LVT viscometer once the product had been conditioned for at least two hours at 25°C. The product clarity was measured at 600 nm using a spectrophotometer. The clarity measurements are reported as % transmittance (% T) with 100% being perfect clarity.

**Table 9: Viscosity and Transmittance of 0.2 wt% HEC in Lysol All-Purpose Cleaner**

| Thickener | HE-MS | Viscosity mPas(cPs) | T (%) |
|---|---|---|---|
| Control - Polymer-Free | - | 9 | 96.9 |
| Invention Example 8 | 0.9 | 44 | 96.0 |
| Natrosol 250HHR CS | 2.5 | 18 | 98.9 |
| Lysol All-Purpose Cleaner as. received | - | 3 | 99.0 |

**Exainple 24**

Pharmaceuticals

Low HE-MS, water soluble HEC excipients provide superior tablet hardness. HEC is used in the pharmaceutical industry as an excipient to provide a swellable diffusion barrier in controlled release applications. The gel matrix it forms limits the diffusion of aqueous fluids into a system and dissolved actives out of the system.

HEC has some unique modified release properties not duplicated by hydroxypropylmethyl cellulose (HPMC) and hydroxypropyl cellulose (HPC). However, current knowledge is that commercial grades of HEC show significantly inferior compression properties when compared to HPMC and HPC. The poor compactibility of this polymer generally makes the polymer suitable for only wet granulation processing, rather than direct compression processing which is frequently the industry preference.

In order to improve this limitation, scientists at Astra Zeneca in International Patent Application, WO 02/19990 A1 describe a procedure whereby HEC is purified by dissolution in water before precipitation via addition of organic solvent. The precipitate is washed and then milled in a specific manner. The purified HEC has markedly improved tablet compactibility.

In accordance with the present invention is the use of a low HE-MS, water soluble HEC material that is highly compressible for making direct compressible tablets for use in compaction applications such as sustained release tablets for pharmaceutical, household, and agricultural applications.

Table 10 shows the strength of pure polymer tablets (with 1% stearic acid for lubrication) made from low HE-MS HEC and commercial Natrosol 250 HHX Pharm HEC. Water soluble HEC with HE-MS 1.1 achieves a 9-fold increase in tablet hardness as compared to regular Natrosol 250 HHX Pharm. In a typical modified release formulations, these materials all showed excellent direct compression performance and drug release kinetics as compared to commercial Natrosol 250 HHX Pharm.

**Table 10: Hardness of 99 wt% HEC With 1 wt% Stearic Acid Tablets**

| Water Soluble HEC. | HE-MS | 1 wt% Solution Viscosity (cps) | Mean Particle Diameter (um) | Tablet Hardness (kP) |
|---|---|---|---|---|
| Invention Example 13 | 1.1 | 27,200 | 121 | 24.6 |
| Natrosol 250 HHX | 2.4 | 3,950 | 105 | 2.7 |

## Claims

1. A slurry process for making a composition comprising hydroxyethylcellulose (HEC) having hydroxyethyl groups that are uniformly distributed on the cellulose backbone wherein the water solubility is greater than 90 % and the hydroxyethyl molar substitution (HE-MS) is in the range of 0.7 to 1.3, said process comprising

   A) mixing and reacting cellulose, water and alkali in an organic solvent for a sufficient time and at a sufficient temperature in order to form an alkali cellulose mixture, wherein the water to anhydroglucose (AGU) molar ratio is in the range of 5 to 35 and the alkali to AGU molar ratio is greater than 1.6,
   B) adding a sufficient amount of ethylene oxide to produce the desired HE-MS
   C) adding a sufficient amount of acid continuously in order to reduce the alkali to AGU molar ratio to less than 0.4 and preferably greater than 0.04, while reacting the ethylene oxide with the alkali cellulose at a sufficient temperature and for a sufficient time to form a water soluble HEC product with a hydroxyethyl molar substitution in the range of 0.7 to 1.3 and an unsubstituted trimer ratio (U3R) of equal to or less than 0.21.

2. The slurry process of claim 1, wherein the organic solvent is selected from the group consisting of ethanol, isopropanol, tert-butanol, acetone, methyl ethyl ketone, and dimethoxyethane and mixtures thereof.

3. The slurry process of claim 1, wherein the alkali is selected from the group consisting of lithium hydroxide, sodium hydroxide, potassium hydroxide, and mixtures thereof.

4. The slurry process of claim 1, wherein the cellulose is selected from the group consisting of cotton linters, wood pulps, and mixtures thereof.

5. The slurry process of claim 1, wherein the HEC composition is further reacted with at least one other derivatizing reagent to form a modified hydroxyethylcellulose composition.

6. The slurry process of claim 5, wherein the derivatizing reagent is selected from the group consisting of nonionic, cationic, anionic organic compounds and mixtures thereof.

7. The slurry process of claim 6 wherein the organic compounds are selected from the group consisting of halides, epoxides, glycidyl ethers, carboxylic acids, isocyanates, and mixtures thereof.

8. The slurry process of claim 1 or 5, wherein the HEC or modified HEC composition is further reacted with a viscosity reducing agent.

9. The slurry process of claim 8, wherein the viscosity reducing agent is selected from the group consisting of peroxides, persulfates, peracids, cellulolytic enzymes, salts of halide oxo acids, oxygen, and ozone.

## Patentansprüche

1. Aufschlämmungsverfahren zur Erzeugung einer Zusammensetzung, umfassend Hydroxyethylcellulose (HEC) mit Hydroxyethylgruppen, die gleichmäßig am Cellulose-Rückgrat verteilt sind, worin die Wasserlöslichkeit größer als 90% ist und die molare Hydroxyethylsubstitution (HE-MS) im Bereich von 0,7 bis 1,3 ist, wobei das Verfahren enthält:

   A) Mischen und Reaktion von Cellulose, Wasser und Alkali in einem organischen Lösungsmittel für eine ausreichende Zeit und eine ausreichende Temperatur, zur Bildung einer Alkalicellulosemischung, worin das molare Verhältnis von Wasser zu Anhydroglycose (AGU) im Bereich von 5 bis 35 ist und das molare Verhältnis von Alkali zu AGU größer als 1,6 ist,
   B) Zugabe einer ausreichenden Menge an Ethylenoxid, zur Erzeugung der gewünschten HE-MS,
   C) kontinuierliche Zugabe einer ausreichenden Menge von Säure, zur Reduktion des molaren Verhältnisses von Alkali zu AGU auf weniger als 0,4 und bevorzugt mehr als 0,04, während das Ethylenoxid mit der Alkalicellulose bei einer ausreichenden Temperatur und für eine ausreichende Zeit reagiert wird, zur Bildung eines wasserlöslichen HEC-Produktes mit einer molaren Hydroxyethylsubstitution im Bereich von 0,7 bis 1,3 und einem unsubstituierten Trimerverhältnis (U3R) von gleich oder weniger als 0,21.

2. Aufschlämmungsverfahren nach Anspruch 1, worin das organische Lösungsmittel ausgewählt ist aus der Gruppe bestehend aus Ethanol, Isopropanol, tert-Butanol, Aceton, Methyethylketon und Dimethoxyethan und Mischungen davon.

3. Aufschlämmungsverfahren nach Anspruch 1, worin das Alkali ausgewählt ist aus der Gruppe bestehend aus Lithiumhydroxid, Natriumhydroxid, Kaliumhydroxid und Mischungen davon.

4. Aufschlämmungsverfahren nach Anspruch 1, worin die Cellulose ausgewählt ist aus der Gruppe bestehend aus Baumwolllintern, Holzpulpen und Mischungen davon.

5. Aufschlämmungsverfahren nach Anspruch 1, worin die HEC-Zusammensetzung weiterhin mit zumindest einem anderen Derivatisierungsreagens reagiert wird, zur Bildung einer modifizierten Hydroxyethylcellulose-Zusammensetzung.

6. Aufschlämmungsverfahren nach Anspruch 5, worin das Derivatisierungsreagens ausgewählt ist aus der Gruppe bestehend aus nichtionischen, kationischen, anionischen organischen Verbindungen und Mischungen davon.

7. Aufschlämmungsverfahren nach Anspruch 6, worin die organischen Verbindungen ausgewählt sind aus der Gruppe bestehend aus Halogeniden, Epoxiden, Glycidylethern, Carbonsäuren, Isocyanaten und Mischungen davon.

8. Aufschlämmungsverfahren nach Anspruch 1 oder 5, worin das HEC oder die modifizierte HEC-Zusammensetzung weiterhin mit einem Viskositätsreduktionsmittel reagiert wird.

9. Aufschlämmungsverfahren nach Anspruch 8, worin das Viskositätsreduktionsmittel ausgewählt ist aus der Gruppe bestehend aus Peroxiden, Persulfaten, Persäuren, cellulolytischen Enzymen, Salzen von Halogenid-Oxosäuren, Sauerstoff und Ozon.

## Revendications

1. Procédé en suspension pour fabriquer une composition comprenant de l'hydroxyéthylcellulose (HEC) ayant des

groupes hydroxyéthyls qui sont uniformément distribués sur la chaîne de cellulose dans lequel la solubilité de l'eau est plus grande que 90% et la substitution molaire hydroxyéthyl (HE-MS) est comprise dans le domaine de 0,7 à 1,3, ledit procédé comprenant :

A) le mélange et la réaction de la cellulose, de l'eau et des alcalins dans un solvant organique pendant un temps suffisant et à une température suffisante afin de former un mélange de cellulose alcaline, dans lequel le rapport molaire de l'eau à l'anhydroglucose (AGU) est compris dans le domaine entre 5 et 35 et le rapport molaire de l'alcalin à l'AGU est plus grand que 1,6,
B) l'ajout d'une quantité suffisante d'oxyde d'éthylène pour produire le HE-MS désiré,
(C) l'ajout en continu d'une quantité suffisante d'acide afin de réduire le rapport molaire de l'alcalin à l'AGU à une valeur inférieure 0,4 et préférentiellement à une valeur supérieure à 0,04, tandis que l'on fait réagir l'oxyde d'éthylène avec la cellulose alcaline à une température suffisante et pendant un temps suffisant pour former un produit HEC soluble dans l'eau avec une substitution molaire en hydroxyéthyl dans le domaine de 0,7 à 1,3 et un rapport de trimère non-substitué (U3R) égal ou inférieur à 0,21.

**2.** Procédé en suspension selon la revendication 1, dans lequel le solvant organique est choisi parmi le groupe consistant en l'éthanol, l'isopropanol, le tert-butanol, l'acétone, la cétone éthyle méthyle et le diméthoxyéthane et les mélanges de ceux-ci.

**3.** Procédé en suspension selon la revendication 1, dans lequel l'alcalin est choisi parmi le groupe consistant en l'hydroxyde de lithium, l'hydroxyde de sodium, l'hydroxyde de potassium et les mélanges de ceux-ci.

**4.** Procédé en suspension selon la revendication 1, dans lequel la cellulose est choisi parmi le groupe consistant en des linters de coton, de la pâte de bois et des mélanges de ceux-ci.

**5.** Procédé en suspension selon la revendication 1, dans lequel la composition HEC est en outre mise en réaction avec au moins un autre réactif de dérivatisation pour former une composition d'hydroxyéthylcellulose modifiée.

**6.** Procédé en suspension selon la revendication 5, **caractérisé en ce que** le réactif de dérivatisation est choisi parmi le groupe consistant en des composés organiques non-ionique, cationique, anionique et des mélanges de ceux-ci.

**7.** Procédé en suspension selon la revendication 6, dans lequel les composés organiques sont choisis parmi le groupe consistant en des halogènes, des époxydes, des éthers glycidyliques, des acides carboxyliques, des isocyanates et des mélanges de ceux-ci.

**8.** Procédé en suspension selon la revendication 1 ou 5, dans lequel le HEC ou la composition HEC modifiée est en outre mise en réaction avec un agent réduisant la viscosité.

**9.** Procédé en suspension selon la revendication 8, dans lequel l'agent réduisant la viscosité est choisi parmi le groupe consistant en des peroxydes, des persulfates, des peracides, des enzymes cellulolytiques, des sels d'acides oxo halogénure, de l'oxygène et de l'ozone.

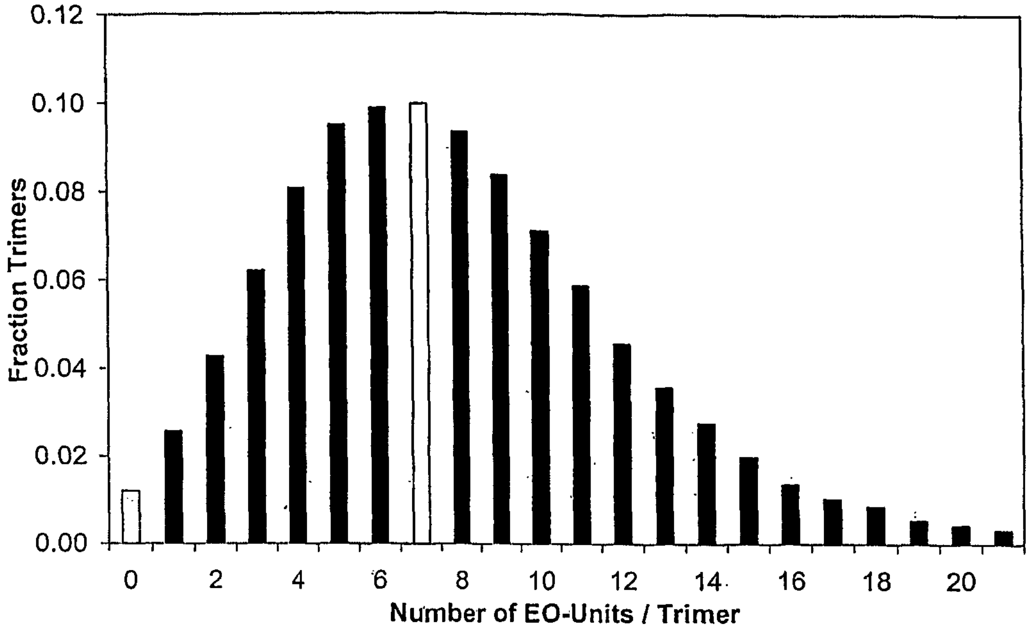
0.12
0.10
0.08
0.06
0.04
0.02
0.00
Fraction Trimers
0
2
4
6
8
10
12
14
16
18
20
Number of EO-Units / Trimer

Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2572039 A
- US 2682535 A
- US 2744894 A
- US 3131177 A
- US 3131176 A
- CA 1014289
- US 20050139130 A1
- US 35362106 A
- US 4228277 A
- US 4826970 A
- US 4904772 A
- US 4663159 A
- WO 0219990 A1


### Non-patent literature cited in the description


- **F.-G. HANISCH.** *Biological Mass Spectrometry,* 1994, vol. 23, 309-312
- **B. LINDBERG ; U. LINDQUIST ; O. STENBERG.** *Carbohydrate Research,* 1987, vol. 170, 207-214
- **P.W. ARISZ ; J.A. LOMAX ; J.J. BOON.** *Carbohydrate Research,* vol. 243 (1993), 99-114